(19) Europäisches Patentamt
European Patent Office
Office européen des brevets

(11) Veröffentlichungsnummer: **0 491 239 B1**

(12) **EUROPÄISCHE PATENTSCHRIFT**

(45) Veröffentlichungstag der Patentschrift: **26.04.95**

(51) Int. Cl.[6]: **C07C 45/50**, B01J 31/24

(21) Anmeldenummer: **91120870.0**

(22) Anmeldetag: **05.12.91**

(54) **Verfahren zur Herstellung von Aldehyden.**

(30) Priorität: **17.12.90 DE 4040315**

(43) Veröffentlichungstag der Anmeldung:
**24.06.92 Patentblatt 92/26**

(45) Bekanntmachung des Hinweises auf die Patenterteilung:
**26.04.95 Patentblatt 95/17**

(84) Benannte Vertragsstaaten:
**AT BE CH DE DK ES FR GB IT LI LU NL SE**

(56) Entgegenhaltungen:
**EP-A- 0 133 127**
**EP-A- 0 279 018**
**EP-A- 0 375 573**

(73) Patentinhaber: **HOECHST AKTIENGESELL-SCHAFT**
**D-65926 Frankfurt (DE)**

(72) Erfinder: **Herrmann, Wolfgang, Prof. Dr. Dipl.-Chem.**
**Gartenstrasse 69**
**W-8050 Freising (DE)**
Erfinder: **Kohlpaintner, Christian, Dipl.-Chem.**
**Kuglmoosstrasse 40**
**W-8209 Stephanskirchen (DE)**
Erfinder: **Bahrmann, Helmut, Dr. Dipl.-Chem.**
**Rostrasse 48**
**W-4236 Hamminkeln (DE)**

EP 0 491 239 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft ein Verfahren zur Herstellung von Aldehyden durch Hydroformylierung von Olefinen in Gegenwart wasserlöslicher Rhodium-Komplexkatalysatoren.

Es ist bekannt, durch Umsetzung von Olefinen mit Kohlenmonoxid und Wasserstoff (Hydroformylierung) Aldehyde und Alkohole herzustellen, die ein Kohlenstoffatom mehr als das Ausgangsolefin enthalten. Die Reaktion wird durch Hydridometallcarbonyle, vorzugsweise solcher der Metalle der VIII. Gruppe des Periodensystems, katalysiert. Neben Kobalt, das als Katalysatormetall in großem Umfang technische Anwendung findet, gewinnt in letzter Zeit Rhodium zunehmend Bedeutung. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen in Gegenwart von Rhodium-Katalysatoren deutlich niedriger als bei Anwendung von Kobalt-Katalysatoren.

Bei den in der Technik eingeführten Verfahren wird der Rhodium-Katalysator in Form modifizierter Hydridorhodiumcarbonyle eingesetzt, die zusätzliche Liganden, insbesondere tertiäre, organische Phosphane oder Phosphite enthalten. Meist liegen die Liganden im Überschuß vor, so daß das Katalysatorsystem aus Komplexverbindung und freiem Ligand besteht. Der Einsatz der beschriebenen Rhodium-Katalysatoren ermöglicht es, die Hydroformylierungsreaktion bei Drücken unter 30 MPa durchzuführen.

Schwierigkeiten verursachen bei diesem Verfahren jedoch die Abtrennung der Reaktionsprodukte und die Wiedergewinnung der im Reaktionsprodukt homogen gelösten Katalysatoren. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 8 Kohlenstoffatomen im Molekül, beschritten werden. Außerdem hat sich gezeigt, daß die thermische Belastung des Destillationsgutes auch zu erheblichen Katalysatorverlusten durch Zersetzung der Rhodium-Komplexverbindungen führt.

Die geschilderten Mängel werden durch Anwendung von Katalysatorsystemen vermieden, die in Wasser löslich sind. Derartige Katalysatoren sind z.B. in der DE-PS 26 27 354 beschrieben. Die Löslichkeit der Rhodium-Komplexverbindungen wird hierbei durch Verwendung sulfonierter Triarylphosphane als Komplexbestandteil erreicht. Die Abtrennung des Katalyators vom Reaktionsprodukt nach Beendigung der Umsetzung erfolgt bei dieser Verfahrensvariante einfach durch Trennung von wäßriger und organischer Phase, d.h. ohne Destillation und damit ohne zusätzliche thermische Verfahrensschritte. Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Als Komplexbestandteile wasserlöslicher Rhodium-Komplexverbindungen werden vorzugsweise sulfonierte Triarylphosphane und daneben auch carboxylierte Triarylphosphane eingesetzt.

Die bekannten Zweiphasenverfahren haben sich in technischem Maßstab sehr gut bewährt. Dennoch bemüht man sich um eine weitere Vervollkommnung des Prozesses. So versucht man, die Aktivität der Katalysatoren durch Modifizieren der Komplexliganden zu erhöhen und ihre Wirksamkeit zu verlängern, um den spezifischen Katalysatorbedarf - sowohl Rhodium als auch Ligand - und damit die Produktionskosten weiter zu senken. Wirtschaftliche Gründe sind auch dafür maßgebend, auf eine deutliche Verminderung des Phosphan/Rhodium-Verhältnisses hinzuarbeiten. Ferner strebt man an, die bisher erzielte hohe Selektivität hinsichtlich der Bildung unverzweigter Aldehyde noch zu verbessern. Hierbei ist zu berücksichtigen, daß jährlich mehrere Millionen Tonnen Hydroformylierungsprodukte hergestellt werden, so daß schon eine geringe Steigerung der Selektivität wirtschaftlich bedeutsame Konsequenzen hat.

Im Falle der homogen gelösten Katalysatoren ist die Verwendung tertiärer Diphosphane als Komplexliganden bekannt. So wird in der EP-A1-0 279 018 der Einsatz von Diphosphanen beschrieben, die sich von Biarylverbindungen, wie Biphenyl, ableiten. Es zeigt sich jedoch, daß die Aktivität dieser Phosphorverbindungen als Bestandteil von Katalysatorsystemen tertiären Monophosphanen, wie Triphenylphosphan, unterlegen ist.

Gegenstand der Erfindung ist es, den Hydroformylierungsprozeß wie vorstehend skizziert zu verbessern, d.h. Katalysatoren zu entwickeln, die bei möglichst niedrigem Ligand/Rhodium-Verhältnis Aktivität und Selektivität bekannter Katalysatoren übertreffen.

Die Erfindung besteht in einem Verfahren zur Herstellung von Aldehyden durch Umsetzung von Monoolefinen, nichtkonjugierten Polyolefinen, Cycloolefinen oder Derivaten dieser Verbindungsklassen mit Kohlenmonoxid und Wasserstoff bei Temperaturen von 20 bis 150 °C und Drücken von 0,1 bis 20 MPa in Gegenwart wasserlöslicher, Phosphane in komplexer Bindung enthaltender Rhodiumverbindungen als Katalysatoren. Es ist dadurch gekennzeichnet, daß als Phosphane von Biarylverbindungen abgeleitete Diphosphane der allgemeinen Formel I

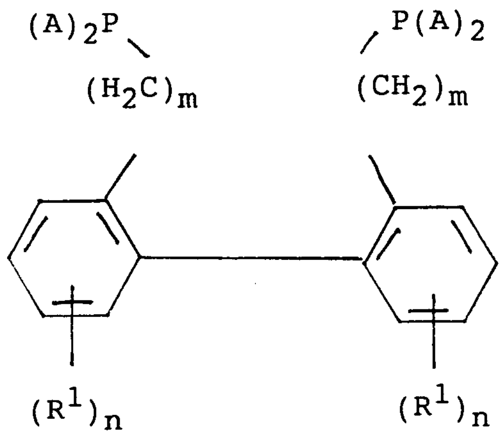

(I)

die durch eine oder mehrere Sulfonsäuregruppen substituiert sind, eingesetzt werden, und in Formel I A für gleiche oder verschiedene Alkyl-, Cycloalkyl-, Phenyl-, Tolyl- oder Naphthylreste steht, $R^1$ gleich oder verschieden ist und Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen oder einen anellierten Benzolring bedeutet, m gleich oder verschieden und eine ganze Zahl von 0 bis 5 ist und n ebenfalls gleich oder verschieden ist und für ganze Zahlen von 0 bis 4 steht.

Die nach dem neuen Verfahren als Katalysatoren eingesetzten wasserlöslichen Rhodium-Diphosphan-Komplexverbindungen zeichnen sich durch eine bemerkenswert hohe Wirksamkeit, bestimmt durch die beiden Kriterien "Aktivität" A, nämlich

$$A = \frac{\text{mol Aldehyd}}{\text{mol Rh} \bullet \text{min}}$$

und "Produktivität" P, nämlich

$$P = \frac{\text{g Aldehyd}}{\text{ml Katalysatorlösung} \bullet \text{h}}$$

aus. Die mit den Verfahren des Standes der Technik erreichten Werte für diese beiden Größen werden von der erfindungsgemäßen Arbeitsweise erheblich übertroffen. Die Bildung normaler Aldehyde nimmt weiter zu und der Austrag von Edelmetall und Phosphan mit dem Reaktionsprodukt wird vermindert. Überdies erzielt man diese Ergebnisse mit Katalysatoren, die ein deutlich geringeres Ligand/Rhodium-Verhältnis aufweisen, als die bisher eingesetzten. Diese und andere, für die Durchführung des Prozesses im technischen Maßstab sehr wertvollen Befunde waren weder aus theoretischen Überlegungen ableitbar noch aus der praktischen Erfahrung vorherzusehen.

Die für das neue Verfahren als Katalysatorbestandteil charakteristischen sulfonierten Diphosphane kann man aus Biarylen, die nach bekannten Synthesen, z.B. durch Kupplung von Arylgrignardreagenzien mit Arylhalogeniden zugänglich sind, herstellen. Auch die Einführung des phosphororganischen Restes $-(H_2C)_mP(A)_2$, in das Biarylmolekül erfolgt nach konventionellen Methoden, z.B. durch Reaktion einer Phosphorverbindung der allgemeinen Formel $X-(H_2C)_mP(A)_2$, in der X für ein Halogenatom steht mit dem Biaryl in Gegenwart eines Protonen abspaltenden Reagenzes wie Natriumamid oder Butyllithium. Im letzten Reaktionsschritt sulfoniert man das Diphosphan mit Oleum, also einer Lösung von Schwefeltrioxid in Schwefelsäure bei Temperaturen von 0 bis 60 °C. Die Isolierung des Sulfonierungsproduktes aus der sauren, mit Wasser verdünnten Lösung erfolgt entsprechend dem Stand der Technik z.B. durch Extraktion mit der Lösung eines wasserunlöslichen Amins in einem wasserunlöslichen organischen Lösungsmittel.

Bevorzugt werden nach der erfindungsgemäßen Arbeitsweise sulfonierte Diphosphane, die sich von Biarylen der allgemeinen Formel I ableiten, in der A gleich oder verschieden ist und einen Phenyl-, Tolyl- oder Naphthylrest bedeutet, $R^1$ ebenfalls gleich oder verschieden ist und für Wasserstoff, für einen Methyl-, Isopropyl-, Isobutyl-, t-Butyl-, Phenyl- oder Naphthylrest oder einen anellierten Benzolring (so daß sich eine Naphthylstruktur ausbildet) steht, m 1 und n 0 oder 1 ist.

Große Bedeutung im Rahmen des beanspruchten Prozesses haben auch sulfonierte Diphosphane, deren Biarylgerüst durch Reste $R^1$ in 6- und 6'-Stellung substituiert sind. Die Anwesenheit dieser Reste hindert die Rotation der beiden substituierten Phenylreste. Daher können Rhodium-Komplexverbindungen, die solche Moleküle als Liganden enthalten, als Katalysatoren für enantioselektive Reaktionen eingesetzt werden.

Beispiele für Diphosphane, die mit Erfolg im neuen Verfahren verwendet werden, sind die durch Sulfonierung von 2,2'-Bis(diphenylphosphanomethyl)-biphenyl (nachfolgend BISBIS genannt) und von 2-(Diphenylphosphanomethyl)-1-[2-(diphenylphosphanomethyl)phenyl]-naphthalin (nachfolgend PHENAPS genannt) erhaltenen Produkte.

Es ist nicht erforderlich, die Diphosphane als einheitliche Verbindungen einzusetzen. Gemische sulfonierter Diphosphane, die sich von Biarylverbindungen unterschiedlicher Struktur ableiten sind ebenso geeignet wie Gemische aus gleiche oder verschiedene Phosphanreste enthaltenden Biarylen unterschiedlicher Sulfonierungsstufe. Schließlich ergeben auch Gemische aus sulfonierten Mono- und Diphosphanen in Kombination mit Rhodium sehr wirksame Katalysatoren. So haben sich z.B. Mischungen aus BISBIS und Triphenylphosphan-trisulfonat-Na (im folgenden TPPTS genannt) bewährt.

Es hat sich bewährt, Rhodium und sulfoniertes Diphosphan nicht in stöchiometrischem Verhältnis, also entsprechend der chemischen Zusammensetzung der Rhodium-Komplexverbindung zu verwenden, die sich im Verlauf der Hydroformylierungsreaktion bildet, sondern das Diphosphan im Überschuß einzusetzen. Dabei kann man das Verhältnis von Rhodium und Diphosphan in weiten Grenzen variieren und je mol Rhodium etwa 1 bis 130 mol Diphosphan anwenden. Bevorzugt wird ein Verhältnis von Rhodium zu Diphosphan im Bereich von 1 : 2 bis 1 : 25 und insbesondere 1 : 2 bis 1 : 10 mol.

Rhodium gelangt entweder als Metall oder als Verbindung zum Einsatz. In metallischer Form verwendet man es entweder als feinverteilte Partikel oder in dünner Schicht auf einem Träger wie Aktivkohle, Calciumcarbonat, Aluminiumsilikat, Tonerde, niedergeschlagen. Als Rhodiumverbindungen kommen solche Substanzen in Betracht, die wasserlöslich sind oder unter den Reaktionsbedingungen wasserlöslich werden. Geeignet sind die verschiedenen Rhodiumoxide, Salze anorganischer Wasserstoff- und Sauerstoffsäuren, sowie Salze aliphatischer Mono- und Polycarbonsäuren. Beispiele für Rhodiumsalze sind Rhodiumnitrat, Rhodiumsulfat, Rhodiumacetat, Rhodium-2-ethylhexanoat, Rhodiummalonat. Rhodiumhalogenverbindungen kommen wegen des korrosiven Verhaltens der Halogenidionen dagegen weniger in Betracht. Weiterhin können Rhodiumcarbonylverbindungen wie $Rh_3(CO)_{12}$ oder $Rh_6(CO)_{16}$ oder Komplexsalze des Rhodiums, z.B. Cyclooctadienylrhodiumverbindungen eingesetzt werden. Bevorzugt werden Rhodiumoxid und insbesondere Rhodiumacetat und Rhodium-2-ethylhexanoat. Es ist anzunehmen, daß sich in Gegenwart von Wassergas unter den Bedingungen der Hydroformylierungsreaktion wasserlösliche Rhodium-Komplexverbindungen bilden, die Kohlenmonoxid und Diphosphan als Liganden enthalten. Sie ergeben zusammen mit dem im Wasser gelösten Diphosphan das Katalysatorsystem.

Die Katalysatorlösung wird aus den Komponenten entweder im Hydroformylierungsreaktor oder, im voraus, in einer separaten Vorrichtung hergestellt und darauf dem Hydroformylierungsreaktor zugeleitet.

Die Konzentration des Rhodiums in der wäßrigen Katalysatorlösung beträgt 20 bis 1000 Gew.-ppm (bezogen auf die Lösung), vorzugsweise 100 bis 600 Gew.-ppm und insbesondere 200 bis 400 Gew.-ppm.

Die Umsetzung des Olefins mit Kohlenmonoxid und Wasserstoff erfolgt bei Drücken von 0,1 bis 30 MPa, vorzugsweise 1 bis 12 MPa und insbesondere 3 bis 7 MPa. Die Zusammensetzung des Synthesegases, d.h. das Volumenverhältnis von Kohlenmonoxid und Wasserstoff kann sich über weite Bereiche erstrecken und z.B. zwischen 1 : 10 bis 10 : 1 variiert werden. Im allgemeinen setzt man Gasgemische ein, in denen das Volumverhältnis von Kohlenmonoxid und Wasserstoff etwa 1 : 1 ist oder von diesem Wert in der einen oder in der anderen Richtung nur wenig abweicht.

Die Reaktionstemperatur liegt zwischen 20 und 150 °C, bevorzugt werden 80 bis 140 °C und insbesondere 100 bis 125 °C.

Die Umsetzung der in flüssiger und gasförmiger Phase vorliegenden Reaktionspartner erfolgt in konventionellen Reaktoren. Der Ablauf der Umsetzung wird maßgeblich dadurch beeinflußt, daß die wäßrige Katalysatorlösung mit dem flüssigen oder gasförmigen, hydrophoben Olefin und dem Synthesegas gesättigt werden muß. Daher ist es erforderlich, eine möglichst große Berührungsfläche zwischen den Phasen zu erzeugen. Es hat sich bewährt, den flüssigen Reaktorinhalt - Katalysatorlösung, gegebenenfalls flüssiges Olefin und Reaktionsprodukt - intensiv zu rühren und die gasförmigen Reaktionspartner - Synthesegas und

gegebenenfalls Olefin - über Verteilungsvorrichtungen der flüssigen Phase zuzuführen. Es hat sich sehr bewährt, den Anteil der organischen Phase im Reaktionsgemisch gering zu halten. Überraschenderweise trägt die organische Phase zur Löslichkeit der Reaktanten in der wäßrigen Phase nicht bei und es wird vermieden, daß das Reaktionsprodukt unerwünschte Nebenreaktionen eingeht, die bei zunehmender Verweilzeit des Produktes im Reaktor nicht auszuschließen sind. Dementsprechend stellt man ein Volumverhältnis von wäßriger zu organischer Phase von 1 : 1 bis 100 : 1, vorzugsweise 10 : 1 bis 100 : 1 ein. Zu diesem Zweck kann man kontinuierlich einen entsprechenden Teil des Reaktionsgemisches aus dem Reaktor ausschleusen, wäßrige und organische Phase voneinander trennen und die wäßrige Phase in den Reaktor zurückführen. Die Umsetzung kann absatzweise oder, bevorzugt, kontinuierlich durchgeführt werden.

Das erfindungsgemäße Verfahren ist mit Erfolg auf die Umsetzung von Monoolefinen, nicht konjugierten Polyolefinen, cyclischen Olefinen und Derivaten dieser ungesättigten Verbindungen anwendbar. Die Olefine können geradkettig oder verzweigt, die Doppelbindungen end- oder innenständig sein. Beispiele für Olefine, die in dem neuen Prozeß verwendet werden können sind Ethylen, Propylen, Buten-1, Buten-2, Penten-1, 2-Methylbuten-1, Hexen-1, Hexen-2, Hepten-1, Octen-1, Octen-3, 3-Ethylhexen-1, Decen-1, Undecen-3, 4,4-Dimethylnonen-1, Dicyclopentadien, Vinylcyclohexen, Cyclooctadien, Styrol. Derivate der genannten Olefinarten, die nach der beanspruchten Arbeitsweise hydroformyliert werden können sind z.B. Alkohole, Aldehyde, Carbonsäuren, Ester, Nitrile und Halogenverbindungen, Allylalkohol, Acrolein, Methacrolein, Crotonaldehyd, Methylacrylat, Ethylcrotonat, Diethylfumarat, Diethylmaleinat, Acrylnitril. Mit besonderem Erfolg wird das Verfahren zur Hydroformylierung von Olefinen und Olefinderivaten mit 2 bis 12 Kohlenstoffatomen eingesetzt.

Die folgenden Beispiele erläutern die Erfindung.

Beispiele 1 - 4

Einem mit Rührer ausgerüsteten 0,2 l Edelstahlautoklav werden Propylen und ein aus gleichen Volumenteilen bestehendes $CO/H_2$-Gemisch in einer solchen Menge zugeleitet, daß je Stunde 10 Nl Abgas aus dem Reaktor entnommen werden können. Gleichzeitig werden je Stunde 300 ml wäßrige Katalysatorlösung im Kreis durch den Reaktor geführt. Der Katalysator besteht aus 0,09 g Rhodium (als Acetat) und 5,89 mmol P(III) (in Form von BISBIS), die in entgastem und mit Stickstoff gesättigtem Wasser zu 300 ml Lösung gelöst wurden. Das Molverhältnis von Phosphor zu Rhodium beträgt 6,7 : 1, entsprechend einem Ligand/Rhodium-Verhältnis von 3,4 : 1. Die Umsetzung der Reaktionspartner erfolgt bei einer Temperatur von 122 °C und einem Druck von 5 MPa.

In der nachfolgenden Tabelle 1 sind die Ergebnisse des erfindungsgemäßen Verfahrens (Beispiele 1 bis 3) denen gegenübergestellt, die mit einer Arbeitsweise nach dem Stand der Technik (Katalysator : Rhodium/TPPTS) erhalten werden (Beispiel 4). Die Beispiele 2 und 3 zeigen sehr deutlich, daß die neue Arbeitsweise völlig überraschend eine erhebliche Steigerung des Propyleneinsatzes erlaubt. Unter solchen Reaktionsbedingungen ergeben die bekannten Verfahren mit Rh/TPPTS-Katalysatoren keine oder nur sehr geringe Umsätze.

T a b e l l e

| Versuchsbedingungen | Beispiel 1 | Beispiel 2 | Beispiel 3 | Beispiel 4 (Vergleich) |
|---|---|---|---|---|
| Katalysator | Rh/BISBIS | Rh/BISBIS | Rh/BISBIS | Rh/TPPTS |
| Rhodium/Ligand (mol/mol) | 1:3,4 | 1:3,4 | 1:3,4 | 1:80 |
| Temperatur (°C) | 122 | 123,5 | 122 | 122 |
| Druck (MPa) | 5,0 | 5,0 | 5,0 | 5,0 |
| Propyleneinsatz (g/h) | 39,0 | 91,6 | 110,6 | 37,6 |
| **Versuchsergebnisse** | | | | |
| Umsatz (%) | 79,9 | 57,8 | 47,9 | 41,8 |
| Aktivität $\frac{\text{mol (n+i) Aldehyd}}{\text{mol Rh . min}}$ | 30,25 | 60,5 | 90,4 | 15,8 |
| Produktivität $\frac{\text{g (n+i) Aldehyd}}{\text{ml Kat.-lsg. . h}}$ | 0,412 | 0,824 | 1,16 | 0,213 |
| n-Aldehyd (g/h) | 49,2 | 98,2 | 90,8 | 28,0 |
| n/i-Verhältnis (Gew.-teile) | 97/3 | 97/3 | 96/4 | 94/6 |
| Alkohol (%) | 8,45 | 1,34 | 2,07 | 0,73 |
| Andere (%) | 1,5 | 0,16 | 0,24 | 0,57 |

Beispiele 5 bis 14

Die Beispiele 5 bis 14 betreffen die Hydroformylierung von Propylen in der in den Beispielen 1 bis 4 verwendeten Apparatur. Die Versuchsbedingungen sind nachstehend, die Versuchsergebnisse in Tabelle 2

zusammengestellt.

| Versuchsbedingungen | |
|---|---|
| Katalysator | Rh/BISBIS |
| Rh-Konzentration (ppm, bez. auf Kat.-Lösg.) | 306 |
| Rh/Ligand (mol/mol) | 1 : 3,4 |
| Temperatur (°C) | 125 |
| Druck (MPa) | 5 |

Tabelle 2

| | Beispiele 5 | 6 | 7 | 8 | 9 | 10 | 11 | 12 | 13 | 14 |
|---|---|---|---|---|---|---|---|---|---|---|
| Propyleneinsatz (g/h) | 23,0 | 49,9 | 65,9 | 82,7 | 100,8 | 110,6 | 128,4 | 125,2 | 124,0 | 108,0 |
| Aktivität $\frac{\text{mol (n+i) Aldehyd}}{\text{mol Rh . min}}$ | 27,45 | 42,4 | 54,58 | 67,37 | 64,37 | 90,47 | 90,31 | 46,29 | 31,04 | 97,68 |
| Produktivität $\frac{\text{g (n+i) Aldehyd}}{\text{ml Kat.-lsg. . h}}$ | 0,35 | 0,55 | 0,70 | 0,87 | 0,83 | 1,16 | 1,16 | 0,60 | 0,40 | 1,26 |
| n-Aldehyd (g/h) | 83,48 | 84,59 | 85,46 | 87,58 | 89,73 | 87,64 | 88,0 | 89,35 | 89,00 | 87,37 |
| n/i-Verhältnis (Gew.-Teile) | 95,61 | 97,05 | 96,12 | 95,81 | 95,89 | 95,72 | 92,26 | 96,45 | 96,51 | 96,86 |
| Alkohol (Gew.-%) | 9,82 | 9,25 | 6,12 | 3,36 | 2,13 | 2,37 | 1,85 | 1,70 | 1,72 | 1,37 |

Beispiele 15 bis 19

Die Beispiele 15 bis 19 betreffen die Hydroformylierung von Hexen in der in den Beispielen 1 bis 4 verwendeten Apparatur. Die Versuchsbedingungen sind nachstehend, die Versuchsergebnisse in Tabelle 3

zusammengestellt.

| Versuchsbedingungen | |
|---|---|
| Katalysator | Rh/BISBIS |
| Rh-Konzentration (ppm, bez. auf Kat.-Lösg.) | 306 |
| Rh/Ligand (mol/mol) | 1 : 3,4 |
| Druck (MPa) | 5 |

Tabelle 3

| | Beispiele | | | | |
|---|---|---|---|---|---|
| | 15 | 16 | 17 | 18 | 19 |
| Hexeneinsatz (g/h) | 14,50 | 13,90 | 13,90 | 15,50 | 15,40 |
| Aktivität mol (n+i)-Aldehyd / mol Rh . min | 0,73 | 1,27 | 1,99 | 4,16 | 10,69 |
| Produktivität g (n+i)-Aldehyd / ml Kat.-Lsg . h | 0,004 | 0,007 | 0,011 | 0,023 | 0,058 |
| n-Aldehyd (g/h) | 4,60 | 8,90 | 13,80 | 26,10 | 30,30 |
| n/i-Verhältnis (Gew.-Teile) | 97,12 | 96,23 | 96,00 | 95,38 | 94,56 |

## Patentansprüche

1. Verfahren zur Herstellung von Aldehyden durch Umsetzung von Monoolefinen, nichtkonjugierten Polyolefinen, Cycloolefinen oder Derivaten dieser Verbindungsklassen mit Kohlenmonoxid und Wasserstoff bei Temperaturen von 20 bis 150 °C und Drücken von 0,1 bis 20 MPa in Gegenwart wasserlöslicher, Phosphane in komplexer Bindung enthaltender Rhodiumverbindungen als Katalysatoren, dadurch gekennzeichnet, daß als Phosphane von Biarylverbindungen abgeleitete Diphosphane der allgemeinen Formel I

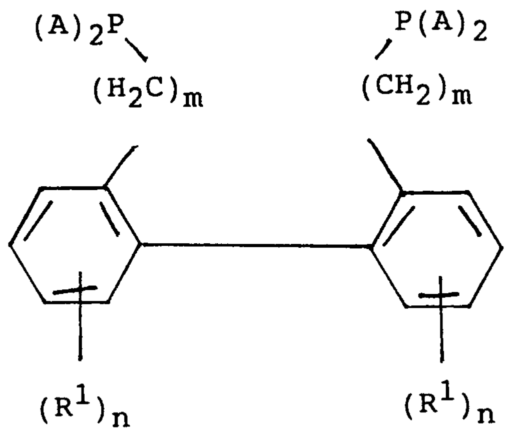

(I)

die durch eine oder mehrere Sulfonsäuregruppen substituiert sind, eingesetzt werden, und in Formel I A für gleiche oder verschiedene Alkyl-, Cycloalkyl-, Phenyl-, Tolyl- oder Naphthylreste steht, $R^1$ gleich oder verschieden ist und Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen oder einen anellierten Benzolring bedeutet, m gleich oder verschieden und eine ganze Zahl von 0 bis 5 ist und n ebenfalls gleich oder verschieden ist und für ganze Zahlen von 0 bis 4 steht.

2. Verfahren nach Anspruch 1, dadurch gekennzeichnet, daß in der allgemeinen Formel I A für einen Phenyl-, Tolyl- oder Naphthylrest steht.

3. Verfahren nach einem oder mehreren der Ansprüche 1 oder 2, dadurch gekennzeichnet, daß in der allgemeinen Formel I $R^1$ für Wasserstoff, für einen Methyl-, Isopropyl-, Isobutyl-, t-Butyl-, Phenyl- oder Naphthylrest oder einen anellierten Benzolring steht.

4. Verfahren nach einem oder mehreren der Ansprüche 1 bis 3, dadurch gekennzeichnet, daß in der allgemeinen Formel I m = 1 ist.

5. Verfahren nach einem oder mehreren der Ansprüche 1 bis 4, dadurch gekennzeichnet, daß n = 0 oder 1 ist.

6. Verfahren nach einem oder mehreren der Ansprüche 1 bis 5, dadurch gekennzeichnet, daß in der allgemeinen Formel I Substituenten $R^1$ (ausgenommen in der Bedeutung anellierter Benzolring) in 6- und 6'-Stellung stehen.

7. Verfahren nach einem oder mehreren der Ansprüche 1 bis 6, dadurch gekennzeichnet, daß je mol Rhodium 1 bis 130 mol Diphosphan eingesetzt werden.

8. Verfahren nach Anspruch 7, dadurch gekennzeichnet, daß je mol Rhodium 2 bis 10 mol Diphosphan eingesetzt werden.

9. Verfahren nach einem oder mehreren der Ansprüche 1 bis 8, dadurch gekennzeichnet, daß die Rhodiumkonzentration in der wäßrigen Katalysatorlösung 20 bis 1000 Gew.-ppm (bezogen auf die Lösung) beträgt.

**10.** Verfahren nach Anspruch 9, dadurch gekennzeichnet, daß die Rhodiumkonzentration in der wäßrigen Katalysatorlösung 200 bis 400 Gew.-ppm (bezogen auf die Lösung) beträgt.

**11.** Verfahren nach einem oder mehreren der Ansprüche 1 bis 10, dadurch gekennzeichnet, daß die Umsetzung bei 20 bis 150 °C erfolgt.

**12.** Verfahren nach Anspruch 11, dadurch gekennzeichnet, daß die Umsetzung bei 100 bis 125 °C erfolgt.

**13.** Verfahren nach einem oder mehreren der Ansprüche 1 bis 12, dadurch gekennzeichnet, daß die Umsetzung bei Drücken von 0,1 bis 30 MPa erfolgt.

**14.** Verfahren nach Anspruch 13, dadurch gekennzeichnet, daß die Umsetzung bei Drücken von 3 bis 7 MPa erfolgt.

**15.** Verfahren nach einem oder mehreren der Ansprüche 1 bis 14, dadurch gekennzeichnet, daß Olefine oder Olefinderivate mit 2 bis 12 Kohlenstoffatomen umgesetzt werden.

**16.** Katalysator zur Hydroformylierung von Monoolefinen, nichtkonjugierten Polyolefinen, Cycloolefinen oder Derivaten dieser Verbindungsklassen enthaltend Rhodium und ein Diphosphan im Molverhältnis 2 : 1 bis 1 : 2, wobei das Diphosphan durch Sulfonierung von Biarylverbindungen der allgemeinen Formel I

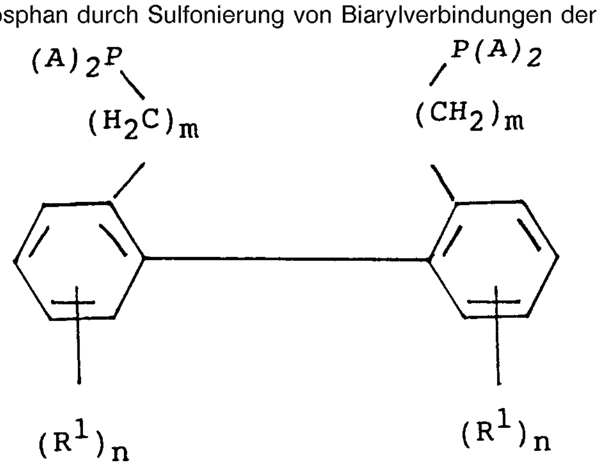

(I)

in der A für Alkyl-, Cycloalkyl-, Phenyl-, Tolyl- oder Naphthylreste steht, $R^1$ gleich oder verschieden ist und Wasserstoff, Alkyl- oder Alkoxyreste mit 1 bis 14 Kohlenstoffatomen, ferner Cycloalkyl-, Aryl- oder Aryloxyreste mit 6 bis 14 Kohlenstoffatomen oder einen anellierten Benzolring bedeutet, m gleich oder verschieden und eine ganze Zahl von 0 bis 5 ist und n ebenfalls gleich oder verschieden ist und für ganze Zahlen von 0 bis 4 steht, erhalten wird.

## Claims

**1.** A process for preparing aldehydes by reacting monoolefins, unconjugated polyolefins, cycloolefins or derivatives of these classes of compounds with carbon monoxide and hydrogen at temperatures of 20 to 150 °C and pressures of 0.1 to 20 MPa in the presence of water-soluble rhodium compounds containing phosphanes in a complex bond as catalysts, characterised in that diphosphanes derived from biaryl compounds and having the general formula I are used as phosphanes,

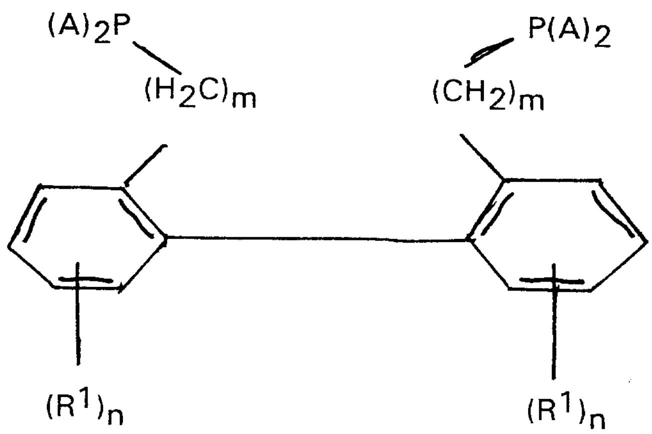

(I)

said diphosphanes being substituted by one or more sulfonic acid groups and in the formula I A stands for the same or different alkyl, cycloalkyl, phenyl, tolyl or naphthyl radicals, $R^1$ is the same or different and denotes hydrogen, alkyl or alkoxy radicals having 1 to 14 carbon atoms, or cycloalkyl, aryl or aroxy radicals having 6 to 14 carbon atoms or a fused benzene ring, m is the same or different and is an integer from 0 to 5 and n is also the same or different and stands for integers from 0 to 4.

**2.** A process according to claim 1, characterised in that in the formula I A stands for a phenyl, tolyl or naphthyl radical.

**3.** A process according to one or both of claims 1 and 2, characterised in that in the general formula I $R^1$ stands for hydrogen, a methyl, isopropyl, isobutyl, t-butyl, phenyl or naphthyl radical or a fused benzene ring.

**4.** A process according to one or more of claims 1 to 3, characterised in that in the general formula I m is equal to 1.

**5.** A process according to one or more of claims 1 to 4, characterised in that in the general formula I n is equal to 0 or 1.

**6.** A process according to one or more of claims 1 to 5, characterised in that in the general formula I substitutes $R^1$ (with the exception of a fused benzene ring) are located in the 6 and 6' positions.

**7.** A process according to one or more of claims 1 to 6, characterised in that 1 to 130 mol of diphosphane are used per mole of rhodium.

**8.** A process according to claim 7, characterised in that 2 to 10 mol of diphosphane are used per mole of rhodium.

**9.** A process according to one or more of claims 1 to 8, characterised in that the rhodium concentration in the aqueous catalyst solution is 20 to 1000 ppm by weight (related to the solution).

**10.** A process according to claim 9, characterised in that the rhodium concentration in the aqueous catalyst solution is 200 to 400 ppm by weight (related to the solution).

**11.** A process according to one or more of claims 1 to 10, characterised in that the reaction takes place at 20 to 150 °C.

**12.** A process according to claim 11, characterised in that the reaction takes place at 100 to 125 °C.

13. A process according to one or more of claims 1 to 12, characterised in that the reaction takes place at pressures of 0.1 to 30 MPa.

14. A process according to claim 13, characterised in that the reaction takes place at pressures of 3 to 7 MPa.

15. A process according to one or more of claims 1 to 14, characterised in that olefins or olefin derivatives having 2 to 12 carbon atoms are reacted.

16. A catalyst for the hydroformylation of monoolefins, unconjugated polyolefins, cycloolefins or derivatives of these classes of compounds containing rhodium and a diphosphane in a molar ratio of 2:1 to 1:2, the diphosphane being obtained by sulfonation of biaryl compounds of the general formula I

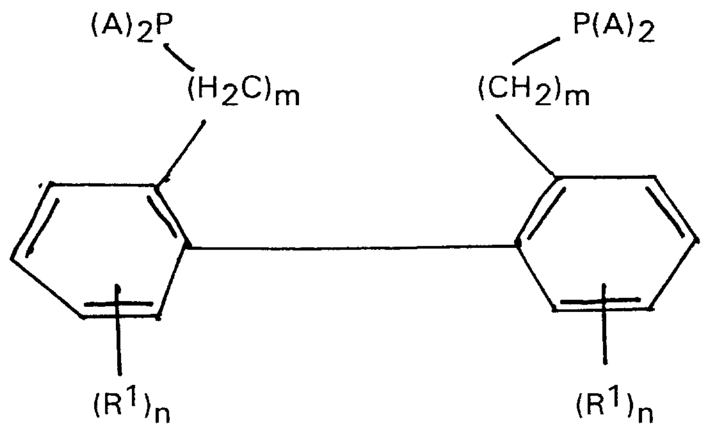

(I)

where A stands for alkyl, cycloalkyl, phenyl, tolyl or naphthyl radicals, $R^1$ is the same or different and stands for hydrogen, alkyl or alkoxy radicals having 1 to 14 carbon atoms, and cycloalkyl, aryl or aryloxy radicals having 6 to 14 carbon atoms or a fused benzene ring, m is the same or different and denotes an integer from 0 to 5 and n is also the same or different and stands for integers from 0 to 4.

**Revendications**

1. Procédé pour la préparation d'aldéhydes par réaction de monooléfines, de polyoléfines non conjugués, de cyclooléfines ou de dérivés de cette classe de composés avec l'oxyde de carbone et l'hydrogène à des températures de 20 à 150 °C et des pressions de 0,1 à 20 MPa en présence de composés du rhodium solubles dans l'eau, contenant des phosphanes en liaison complexe, qui servent de catalyseurs, caractérisé en ce que l'on utilise en tant que phosphanes des diphosphanes dérivant de composés biaryliques et répondant à la formule générale I

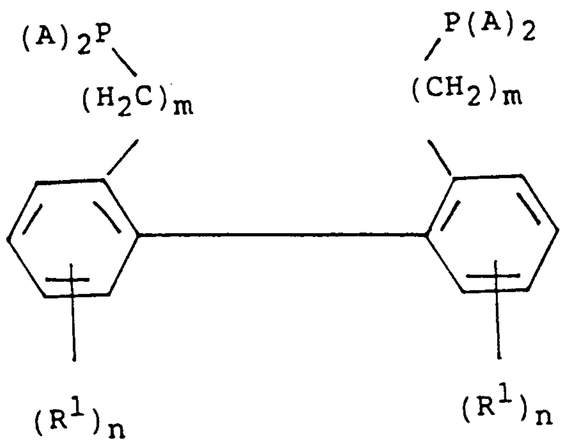

(I)

qui sort substitués par un ou plusieurs groupes acides sulfoniques, les symboles de la formule I ayant les significations suivantes : les symboles A, ayant des significations identiques ou différentes, représentent chacun un groupe alkyle, cycloalkyle, phényle, tolyle ou naphtyle, les symboles $R^1$, ayant des significations identiques ou différentes, représentent chacun l'hydrogène, un groupe alkyle ou alcoxy en $C_1$-$C_{14}$, ou encore un groupe cycloalkyle, aryle ou aroxy en $C_6$-$C_{14}$, ou bien un noyau benzénique condensé, les indices m, ayant des significations identiques ou différentes, représentent chacun un nombre entier allant de 0 à 5 et les indices n, ayant également des significations identiques ou différentes, représentent chacun un nombre entier allant de 0 à 4.

**2.** Procédé selon revendication 1, caractérisé en ce que, dans la formule générale I, A représente un groupe phényle, tolyle ou naphtyle.

**3.** Procédé selon une ou plusieurs des revendications 1 ou 2, caractérisé en ce que, dans la formule générale I, $R^1$ représente l'hydrogène, un groupe méthyle, isopropyle, isobutyle, tert-butyle, phényle ou naphtyle ou un noyau benzénique condensé.

**4.** Procédé selon une ou plusieurs des revendications 1 à 3, caractérisé en ce que, dans la formule générale I, m = 1.

**5.** Procédé selon une ou plusieurs des revendications 1 à 4, caractérisé en ce que n = 0 ou 1.

**6.** Procédé selon une ou plusieurs des revendications 1 à 5, caractérisé en ce que, dans la formule générale I, les substituants $R^1$ (sauf lorsqu'ils représentent un noyau benzénique condensé) sont dans les positions 6 et 6'.

**7.** Procédé selon une ou plusieurs des revendications 1 à 6, caractérisé en ce que l'on utilise de 1 à 130 mol de diphosphane par mole de rhodium.

**8.** Procédé selon revendication 7, caractérisé en ce que l'on utilise de 2 à 10 mol de diphosphane par mole de rhodium.

**9.** Procédé selon une ou plusieurs des revendications 1 à 8, caractérisé en ce que la concentration en rhodium de la solution aqueuse de catalyseur est de 20 à 1 000 ppm en poids (par rapport à la solution).

**10.** Procédé selon revendication 9, caractérisé en ce que la concentration en rhodium de la solution aqueuse de catalyseur est de 200 à 400 ppm en poids (par rapport à la solution).

**11.** Procédé selon une ou plusieurs des revendications 1 à 10, caractérisé en ce que la réaction est effectuée à des températures de 20 à 150 °C.

**12.** Procédé selon revendication 11, caractérisé en ce que la réaction est effectuée à des températures de 100 à 125 °C.

**13.** Procédé selon une ou plusieurs des revendications 1 à 12, caractérisé en ce que la réaction est réalisée sous des pressions de 0,1 à 30 MPa.

**14.** Procédé selon revendication 13, caractérisé en ce que la réaction est réalisée sous des pressions de 3 à 7 MPa.

**15.** Procédé selon une ou plusieurs des revendications 1 à 14, caractérisé en ce que l'on convertit des oléfines ou dérivés d'oléfines en $C_2$-$C_{12}$.

**16.** Catalyseur pour l'hydroformylation des monooléfines, des polyoléfines non conjuguées, des cyclooléfines ou des dérivés de cette classe de composés, ce catalyseur contenant du rhodium et un diphosphane dans un rapport molaire de 2 : 1 à 1 : 2, et le diphosphane ayant été obtenu par sulfonation de biaryles de formule générale I

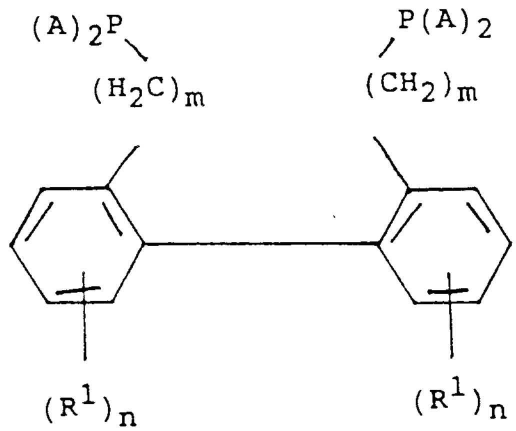

(I)

dans laquelle les symboles A, ayant des significations identiques ou différentes, représentent chacun un groupe alkyle, cycloalkyle, phényle, tolyle ou naphtyle, les symboles $R^1$, ayant des significations identiques ou différentes, représentent chacun l'hydrogène, un groupe alkyle ou alcoxy en $C_1$-$C_{14}$ ou encore un groupe cycloalkyle, aryle ou aryloxy en $C_6$-$C_{14}$, ou un noyau benzénique condensé, les indices m, ayant des significations identiques ou différentes, représentent chacun un nombre entier allant de 0 à 5 et les indices n, ayant également des significations identiques ou différentes, représentent chacun un nombre entier allant de 0 à 4.